(19)

Europäisches Patentamt

European Patent Office

Office européen des brevets

(11) **EP 4 557 407 A1**

(12) **EUROPEAN PATENT APPLICATION**

published in accordance with Art. 153(4) EPC

(43) Date of publication:
**21.05.2025 Bulletin 2025/21**

(21) Application number: **24792169.5**

(22) Date of filing: **22.04.2024**

(51) International Patent Classification (IPC):
$H01M\ 4/62^{(2006.01)}$          $H01M\ 10/42^{(2006.01)}$
$H01M\ 10/0525^{(2010.01)}$     $H01M\ 10/054^{(2010.01)}$

(52) Cooperative Patent Classification (CPC):
Y02E 60/10

(86) International application number:
**PCT/CN2024/089183**

(87) International publication number:
**WO 2024/217586 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.04.2023   CN 202310437795**

(71) Applicant: **SVOLT Energy Technology Co., Ltd.**
**Changzhou, Jiangsu 213200 (CN)**

(72) Inventors:
• **QIAO, Qiqi**
  **Hangzhou, Jiangsu 213200 (CN)**

• **WANG, Pengfei**
  **Hangzhou, Jiangsu 213200 (CN)**
• **SHI, Zetao**
  **Hangzhou, Jiangsu 213200 (CN)**
• **GUO, Feng**
  **Hangzhou, Jiangsu 213200 (CN)**
• **LI, Zitan**
  **Hangzhou, Jiangsu 213200 (CN)**
• **YANG, Hongxin**
  **Hangzhou, Jiangsu 213200 (CN)**

(74) Representative: **Groth & Co. KB**
**P.O. Box 6107**
**102 32 Stockholm (SE)**

(54) **ADDITIVE FOR SUPPLEMENTING LITHIUM OR SODIUM, PREPARATION METHOD THEREFOR AND USE THEREOF**

(57)     The present disclosure relates to the field of batteries, and provides an additive for supplementing lithium or sodium, a preparation method therefor and a use thereof. The additive is mainly prepared from Oxalic acid, a salt, and a catalyst; the salt comprises a lithium salt or a sodium salt; the particle size distribution concentration ratio of the additive for supplementing lithium or sodium satisfies the following expression: $1 \leq (D90-D10)/D50 \leq 100$; the specific surface area of the additive for supplementing lithium or sodium is S, and S and D10, D50 and D90 of the additive for supplementing lithium or sodium satisfy the following expression: $1 \leq (S/((D90-D10)/D50) \leq 100$. The additive provided by the present disclosure is low in decomposition voltage, high in specific capacity, small in particle size, and low in catalyst consumption.

Figure 1

EP 4 557 407 A1

**Description**

**CROSS REFERENCE TO RELATED APPLICATIONS**

The present application claims priority to Chinese Patent Application No. 2023104377951, entitled "ADDITIVE FOR SUPPLEMENTING LITHIUM OR SODIUM, PREPARATION METHOD THEREFOR AND USE THEREOF", and filed to China National Intellectual Property Administration on April 21, 2023, the entire contents of which are incorporated herein by reference.

**TECHNICAL FIELD**

The present disclosure belongs to the field of batteries and relates to an additive for supplementing lithium or sodium and a preparation method and use thereof.

**BACKGROUND**

When lithium ion batteries and sodium ion batteries are charged in the first cycle, a SEI film will form on the surface of the negative electrode, causing irreversible capacity loss of the positive electrode material, thereby affecting the capacity and cycle life of the battery. Therefore, the capacity and cycle performance of the battery can be improved by supplementing lithium or sodium at the positive or negative electrode. Compared with supplement lithium and supplement sodium at the negative electrode, supplement lithium and supplement sodium at the positive electrode is simpler and easier. It does not require additional production processes. It only requires adding lithium supplements or sodium supplements in the positive electrode homogenate. Common positive electrode lithium supplements include $Li_2O$, $Li_2O_2$, $Li_2S$, $Li_3N$, $Li_2NiO_2$, $Li_2CuO_2$, $Li_2MnO_3$, $Li_2C_2O_4$, $Li_5FeO_4$, etc. Common positive electrode sodium supplements include $Na_2O$, $Na_2O_2$, $Na_2S$, $Na_3N$, $Na_2NiO_2$, $Na_2CuO_2$, $Na_2C_2O_4$, $Na_5FeO_4$, etc. The positive electrode lithium supplements that have begun to be commercialized are mainly $Li_2NiO_2$ and $Li_5FeO_4$, and the positive electrode sodium supplements have not yet been commercialized. However, the material stability of $Li_2NiO_2$ is poor and surface coating is required. The lithium supplement efficiency is low and the product is $LiNiO_2$, which has poor structural stability and serious gas production at high temperatures. $Li_5FeO_4$ releases a large amount of oxygen during the first delithiation process, oxidizing the electrolyte and causing gas production in the battery cell. LiOH is easily retained on the surface during the synthesis process, resulting in a homogenous gel jelly. In addition, the synthesis conditions of these two lithium supplements are harsh, and the moisture content in the synthesis process needs to be strictly controlled, resulting in high manufacturing costs. Therefore, it is imperative to develop a low-cost, residue-free, and air-stable lithium and sodium supplement.

Lithium oxalate and sodium oxalate are low-cost, air-stable, acidic lithium and sodium supplements that leave no residue after the first cycle of charging. However, their decomposition voltage is high (4.7V), which does not match the voltage of the current mainstream ternary positive electrode material lithium iron phosphate, and therefore has not been commercialized. In Patent CN114300680A, unmodified lithium oxalate was dissolved in water, then cobalt oxide quantum dot dispersion was slowly dropped into the above solution, and then carbon nanotube dispersion was slowly dropped into the above solution after stirring uniformly to obtain a precursor solution, and then the precursor solution was atomized under the action of an ultrasonic atomizer to finally obtain modified lithium oxalate. The synthesis process of lithium oxalate is relatively complicated. In Patent CN114464909, the prepared catalyst was dispersed in a saturated aqueous solution of lithium oxalate, stirred evenly, ethanol was slowly added to the dispersion, lithium oxalate was precipitated by recrystallization, and then centrifugal drying was carried out to obtain a composite lithium supplement material containing lithium oxalate and the catalyst. In Patent CN110112475A, oxalic acid and sodium carbonate solutions were prepared respectively, then the oxalic acid solution was added slowly to the sodium carbonate solution to form a uniform solution, thenthe mixed solution was added to ethanol to generate a precipitate, filtered, and dried to obtain the final product of sodium oxalate $Na_2C_2O_4$, but the sodium oxalate synthesized by this method had a larger particle size of about 1 $\mu$m.

**SUMMARY OF THE INVENTION**

The present disclosure provides an additive for supplementing lithium or sodium mainly prepared from oxalic acid, a salt and a catalyst;

wherein, the salt comprises: a lithium salt or a sodium salt;
the particle size distribution concentration ratio of the additive for supplementing lithium or sodium satisfies the following formula:

$$1 \leq (D90-D10)/D50 \leq 100;$$

the specific surface area of the additive for supplementing lithium or sodium is S, and S and D10, D50 and D90 of the additive for supplementing lithium or sodium satisfy the following formula:

$$1 \leq (S/((D90-D10)/D50) \leq 100.$$

In some embodiments, D50 of oxalic acid, the salt, the catalyst and the additive for supplementing lithium or sodium are Da, Db, Dc and Dd respectively, and Da, Db, Dc and Dd satisfy the following formula:

$$0.5 \leq (Da+Db)/(Dc+Dd) \leq 200.$$

In some embodiments, a mass ratio of oxalic acid, the salt and the catalyst is (170 to 180): (100 to 220): (5 to 30).

In some embodiments, the additive for supplementing lithium or sodium has a particle size of $0.01\mu m$ to $50\mu m$.

In some embodiments, the additive for supplementing lithium or sodium has a pH of less than 7.

In some embodiments, the additive for supplementing lithium or sodium has a bulk density of 0.5 $g/cm^3$ to 1.5 $g/cm^3$.

In some embodiments, the catalyst comprises at least one of $LiCoO_2$, $LiNi_xCo_yMn_{1-x-y}O_2$, $LiFePO_4$, $LiMn_xFe_{1-x}O_4$, $LiMn_2O_4$, $LiNi_{0.5}Mn_{1.5}O_4$, lithium-rich manganese-based positive electrode, $NiO$, $MnO_2$, $Mn_3O_4$, $CoO$, $Co_3O_4$, $Fe_3O_4$, $MoO_3$, $WO_3$, $Nb_2O_5$, $Mo_2C$, $TaC$, $SiC$, $TiN$, $MoN$, $WN$, $TiB_2$, $WB$, Ketjen black, conductive carbon super-P, acetylene black, CNT, VGCF, polyaniline, polypyrrole, polythiophene and polypyridine; in the $LiNi_xCo_yMn_{1-x-y}O_2$, x+y=1, $0<x\leq1$, $0<y<1$; and in the $LiMn_xFe_{1-x}O_4$, $0\leq x\leq1$.

In some embodiments, the lithium salt comprises: $Li_2CO_3$ and/or LiOH.

In some embodiments, the sodium salt comprises at least one of NaOH, $Na_2CO_3$ or $NaHCO_3$.

The present disclosure also provides a method for preparing the additive for supplementing lithium or sodium above, wherein the method comprises the following steps:

mixing a mixed solution containing oxalic acid, the salt and the catalyst and then sand milling and spray drying.

In some embodiments, the sand milling is carried out for a time period of 15 to 30 minutes.

In some embodiments, the sand milling has a material filling efficiency of 40% to 80%.

In some embodiments, the spray drying is carried out at a pressure of 0.2 to 1.0 MPa.

The present disclosure also provides a positive electrode material comprising the additive for supplementing lithium or sodium above.

The present disclosure also provides a lithium ion or sodium ion battery comprising the positive electrode material above.

## DESCRIPTION OF THE DRAWINGS

In order to more clearly illustrate the technical solutions of the embodiments of the present disclosure, the drawings required for use in the embodiments will be briefly introduced below. It should be understood that the following drawings only illustrate the embodiments of the present disclosure by way of example, and the dimensional ratios in the drawings do not directly correspond to the actual ratios of the embodiments. At the same time, the following drawings only show certain embodiments of the present disclosure and therefore should not be regarded as limiting the scope.

Figure 1 is a SEM image (1,000 times) of the additive for supplementing lithium or sodium provided in Example 1 of the present disclosure.

Figure 2 is a SEM image (20,000 times) of the additive for supplementing lithium or sodium provided in Example 1 of the present disclosure.

Figure 3 is a graph showing the lithium ion battery charging test results.

Figure 4 is a graph showing the sodium ion battery charging test results.

## DETAILED DESCRIPTION

Advantages of the embodiments in the content of the invention will be explained in the following embodiment section of the specification, and some of them are obvious from the specification, or can be obtained through some embodiments of the disclosed embodiments.

The technical solution of the present disclosure is further illustrated below with reference to the drawings and some embodiments.

In order to make the objections, technical solutions and advantages of the present disclosure more clearly understood, the present disclosure is further described in detail below in conjunction with the drawings and embodiments. It should be understood that the embodiments described herein are only used to explain the present disclosure, and are not used to limit the present disclosure. In addition, the technical features involved in each embodiment of the present disclosure described below can be combined with each other as long as there is no conflict between them. Without departing from the principles of the embodiments of the present disclosure, several improvements and modifications may be made, and these improvements and modifications are also considered to be within the protection scope of the embodiments of the present disclosure.

The present disclosure provides an additive for supplementing lithium or sodium mainly prepared from oxalic acid, a salt and a catalyst;

wherein, the salt comprises: a lithium salt or a sodium salt;
the particle size distribution concentration ratio of the additive for supplementing lithium or sodium satisfies the following formula:

$$1 \leq (D90-D10)/D50 \leq 100;$$

the specific surface area of the additive for supplementing lithium or sodium is S, and S and D10, D50 and D90 of the additive for supplementing lithium or sodium satisfy the following formula:

$$1 \leq (S/((D90-D10)/D50) \leq 100.$$

In the additive for supplementing lithium or sodium of in the present disclosure, raw materials are strictly selected and the particle size distribution, specific surface area of the additive and the particle size of the raw materials are strictly designed , so that the additive is low in decomposition voltage, high in specific capacity, small in particle size, and low in catalyst consumption.

The particle size distribution concentration ratio of the additive for supplementing lithium or sodium satisfies the following formula: $1 \leq (D90-D10)/D50 \leq 100$. If the concentration ratio is too high, D90 of the material is too large and there are too many large particles, resulting in less contact with the catalyst and low material capacity. If the concentration ratio is too low, the material particles are too small and side reactions will occur with the electrolyte, resulting in a decrease in battery capacity and a decrease in cycle performance.

The particle size of catalyst is small and the specific surface area is too large, which will easily lead to gelation during the positive electrode homogenization process; the particle size of additive is too small, which is not conducive to the uniform mixing of the additive and the positive electrode material; the specific surface area of the additive is S, and S and D10, D50 and D90 of the additive for supplementing lithium or sodium satisfy the following formula: $1 \leq (S/((D90-D10)/D50) \leq 100$. If the parameter is too large, it means that the specific surface area is too large and the particle size distribution concentration is small, which is not conducive to the mixing and homogenization of additives and positive electrode materials; if the parameter is too small, it means that the specific surface area is too small, the particle size distribution concentration is large, and the material particle size is large, resulting in low capacity utilization.

In some embodiments, D50 of oxalic acid, the salt, the catalyst and the additive for supplementing lithium or sodium are Da, Db, Dc and Dd respectively, and Da, Db, Dc and Dd satisfy the following formula:

$$0.5 \leq (Da+Db)/(Dc+Dd) \leq 200.$$

D50 of oxalic acid, the salt, the catalyst and the additive for supplementing lithium or sodium are Da, Db, Dc and Dd respectively, and Da, Db, Dc and Dd satisfy the following formula: $0.5 \leq (Da+Db)/(Dc+Dd) \leq 200$. This formula must be satisfied between raw materials and finished products. If this parameter is too low, it indicates that the particle size of oxalic acid and lithium salt used is small, and the particle size of the catalyst and the synthesized additive is too large. On the one hand, it is not conducive to uniform mixing during the sand milling process. On the other hand, the particle size of catalyst is large, the catalytic effect is poor, the particle size of additive is large, the path of lithium ion embedding and disembedding is long, and the material rate performance is poor, resulting in the capacity cannot be fully utilized; if this parameter is too high, it indicates that the particle size of oxalic acid and the salt used is large, and the particle size of the catalyst and the additive is small, which is not conducive to uniform mixing during the sand milling process.

In some embodiments, a mass ratio of oxalic acid, the salt and the catalyst is (170 to 180): (100 to 220): (5 to 30), e.g., 170: 220: 5, 172: 200: 10, 174: 180: 14, 176: 160: 20, 178: 140: 25 or 180: 100: 30.

In some embodiments, the additive for supplementing lithium or sodium has a particle size of $0.01 \mu m$ to $50 \mu m$, e.g., $0.01 \mu m$, $0.05 \mu m$, $0.1 \mu m$, $0.5 \mu m$, $1 \mu m$, $5 \mu m$, $10 \mu m$, $15 \mu m$, $20 \mu m$, $25 \mu m$, $30 \mu m$, $35 \mu m$, $40 \mu m$, $45 \mu m$ or $50 \mu m$.

In some embodiments, the additive for supplementing lithium or sodium has a pH of less than 7.

In some embodiments, the additive for supplementing lithium or sodium has a bulk density of 0.5 $g/cm^3$ to 1.5 $g/cm^3$, e.g., 0.5 $g/cm^3$, 0.7 $g/cm^3$, 0.9 $g/cm^3$, 1.1 $g/cm^3$, 1.3 $g/cm^3$, or 1.5 $g/cm^3$.

In some embodiments, the catalyst comprises at least one of $LiCoO_2$, $LiNi_xCo_yMn_{1-x-y}O_2$, $LiFePO_4$, $LiMn_xFe_{1-x}O_4$, $LiMn_2O_4$, $LiNi_{0.5}Mn_{1.5}O_4$, lithium-rich manganese-based positive electrode, $NiO$, $MnO_2$, $Mn_3O_4$, $CoO$, $Co_3O_4$, $Fe_3O_4$, $MoO_3$, $WO_3$, $Nb_2O_5$, $Mo_2C$, $TaC$, $SiC$, $TiN$, $MoN$, $WN$, $TiB_2$, $WB$, Ketjen black, conductive carbon super-P, acetylene black, CNT, VGCF, polyaniline, polypyrrole, polythiophene and polypyridine; in the $LiNi_xCo_yMn_{1-x-y}O_2$, x+y=1, 0<x≤1, 0<y<1; and in the $LiMn_xFe_{1-x}O_4$, 0≤x≤1.

In some embodiments, the lithium salt comprises: $Li_2CO_3$ and/or $LiOH$.

In some embodiments, the sodium salt comprises at least one of $NaOH$, $Na_2CO_3$ or $NaHCO_3$.

The present disclosure also provides a method for preparing the additive for supplementing lithium or sodium above, wherein the method comprises the following steps:

mixing a mixed solution containing oxalic acid, the salt and the catalyst and then sand milling and spray drying.

The method for preparing the additive for supplementing lithium or sodium of the present disclosure is simple and easy, and has important practical significance for accelerating the commercialization of lithium oxalate and sodium oxalate; the additive for supplementing lithium or sodium with excellent performance can be obtained by sand milling and spray drying oxalic acid, lithium salt (sodium salt) and catalyst; the additives synthesized by the method are all nano-scale, and are mixed very evenly with the catalyst, with extremely low manufacturing cost, and are easy to mass-produce and commercialize.

In some embodiments, the sand milling is carried out for a time period of 15 to 30 minutes,eg, 15 minutes, 20 minutes, 25 minutes, or 30 minutes.

In some embodiments, the sand milling has a material filling efficiency of 40% to 80%, eg, 40%, 45%, 50%, 55%, 60%, 65%, 70%, 75%, or 80%.

In some embodiments, the spray drying is carried out at a pressure of 0.2 to 1.0 MPa, eg, 0.2 MPa, 0.4 MPa, 0.6 MPa, 0.8 MPa, or 1.0 MPa.

In some embodiments, the spray drying is followed by stoving.

In some embodiments, the stoving is carried out at a temperature of 95°C to 150°C, e.g., 95°C, 100°C, 105°C, 110°C, 115°C, 120°C, 125°C, 130°C, 135°C, 140°C, 145°C, or 150°C.

In some embodiments, the stoving is carried out for a time period of 5 to 15 h, e.g., 5 h, 6 h, 7 h, 8 h, 9 h, 10 h, 11 h, 12 h, 13 h, 14 h, or 15 h.

The present disclosure also relates to a positive electrode material, comprising the additive for supplementing lithium or sodium above.

The present disclosure also relates to a lithium ion or sodium ion battery comprising the positive electrode material above.

Example

The following are typical but non-limiting examples of the present disclosure.

Example 1

The method for preparing the additive for supplementing lithium provided in this example comprises the following steps:

1. 173g of oxalic acid dihydrate ($H_2C_2O_4·2H_2O$), 115g of lithium hydroxide monohydrate ($LiOH·H_2O$), 7.8g of $Co_3O_4$, and 7.8g of KB (Ketjen black) were dissolved in 400g of water and stirred continuously for 4h;
2. After the reaction was completed, the solution was added to the sand mill for sand milling for 23 minutes, and the material filling efficiency was 60%;
3. The sand-milled material was added into the spray dryer for spraying at a pressure of 0.2 MPa;
4. The sprayed material was dried in an oven at 100°C for 10 hours to obtain the final product.

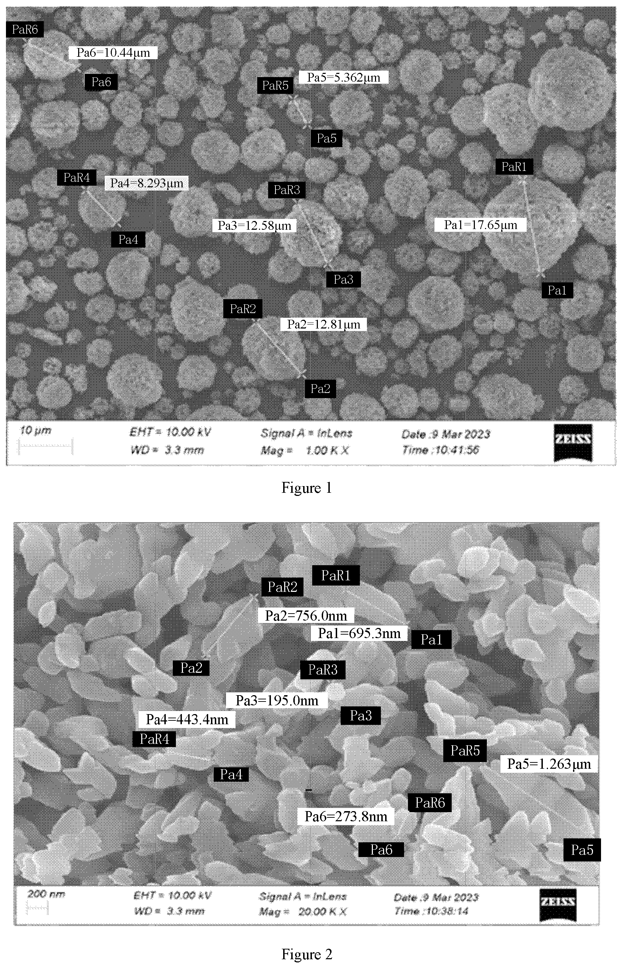

The additive obtained in Example 1 was observed using a scanning electron microscope. Figure 1 is a low magnification electron microscope image. In Figure 1, Pa1 is equal to 17.65$\mu$m, Pa2 is equal to 12.81$\mu$m, Pa3 is equal to 12.58$\mu$m, Pa4 is equal to 8.293$\mu$m, Pa5 is equal to 5.362$\mu$m, and Pa6 is equal to 10.44$\mu$m; Figure 2 is a high magnification electron microscope image. In Figure 2, Pa1 is equal to 695.3nm, Pa2 is equal to 756nm, Pa3 is equal to 195.0nm, Pa4 is equal to 443.4nm, Pa5 is equal to 1.263$\mu$m, and Pa6 is equal to 273.8nm. As can be seen from Figures 1 and 2, the additive is spherical in shape, and the primary particle size is about 400 nm, which is a nanomaterial.

Example 2

The method for preparing the additive for supplementing lithium provided in this example comprises the following steps:

1. 170g of oxalic acid dihydrate ($H_2C_2O_4 \cdot 2H_2O$), 110g of lithium hydroxide monohydrate (LiOH·$H_2O$), 5g of $Co_3O_4$, and 5g of KB (Ketjen black) were dissolved in 400g of water and stirred continuously for 4h;
2. After the reaction was completed, the solution was added to the sand mill for sand milling for 15 minutes, and the material filling efficiency was 40%;
3. The sand-milled material was added into the spray dryer for spraying at a pressure of 0.2 MPa;
4. The sprayed material was dried in an oven at 100°C for 10 hours to obtain the final product.

Example 3

The method for preparing the additive for supplementing lithium provided in this example comprises the following steps:

1. 180g of oxalic acid dihydrate ($H_2C_2O_4 \cdot 2H_2O$), 120g of lithium hydroxide monohydrate (LiOH·$H_2O$), 10g of $Co_3O_4$, and 10g of KB (Ketjen black) were dissolved in 400g of water and stirred continuously for 4h;
2. After the reaction was completed, the solution was added to the sand mill for sand milling for 30 minutes, and the material filling efficiency was 80%;
3. The sand-milled material was added into the spray dryer for spraying at a pressure of 0.2 MPa;
4. The sprayed material was dried in an oven at 100°C for 10 hours to obtain the final product.

Example 4

The method for preparing the additive for supplementing lithium provided in this example comprises the following steps:

1. 176g of oxalic acid dihydrate ($H_2C_2O_4 \cdot 2H_2O$), 118g of lithium hydroxide monohydrate (LiOH·$H_2O$), 8.5g of $Co_3O_4$, and 8.5g of KB (Ketjen black) were dissolved in 400g of water and stirred continuously for 4h;
2. After the reaction was completed, the solution was added to the sand mill for sand milling for 26 minutes, and the material filling efficiency was 75%;
3. The sand-milled material was added into the spray dryer for spraying at a pressure of 0.2 MPa;
4. The sprayed material was dried in an oven at 100°C for 10 hours to obtain the final product.

Example 5

The method for preparing the additive for supplementing lithium provided in this example comprises the following steps:

1. 173g of oxalic acid dihydrate ($H_2C_2O_4 \cdot 2H_2O$), 115g of lithium hydroxide monohydrate (LiOH·$H_2O$), 7.8g of polyaniline, and 7.8g of $LiCoO_2$ were dissolved in 400g of water and stirred continuously for 4h;

Steps 2 to 4 are the same as in Example 1.

Example 6

The method for preparing the additive for supplementing lithium provided in this example comprises the following steps:

1. 173g of oxalic acid dihydrate ($H_2C_2O_4 \cdot 2H_2O$), 115g of lithium hydroxide monohydrate (LiOH·$H_2O$), 7.8g of WN, and 7.8g of $Fe_3O_4$ were dissolved in 400g of water and stirred continuously for 4h;

Steps 2 to 4 are the same as in Example 1.

Example 7

The method for preparing the additive for supplementing sodium provided in this example comprises the following steps:

1. 173g of oxalic acid dihydrate ($H_2C_2O_4 \cdot 2H_2O$), 110g of NaOH, 7.8g of $Co_3O_4$, and 7.8g of KB (Ketjen black) were dissolved in 400g of water and stirred continuously for 4h;

Steps 2 to 4 are the same as in Example 1.

Comparative Example 1

The only difference from Example 1 is that step 2 and step 3 are omitted.

Comparative Example 2

The only difference from Example 7 is that step 2 and step 3 are omitted.

Experimental Example 1

The particle size distribution, specific surface area and pH of the obtained additive were tested using a laser particle size analyzer and a pH meter, and the results are shown in Tables 1 and 2. The D50 of the additive in the examples is between 5.25μm and 8.38 μm, the pH is between 6.54 and 6.85, and the material is acidic, so it will not affect the homogenization of the positive electrode material. Specific surface area is from 37 $m^2$/g to 57$m^2$/g. The additives in the comparative examples were not subjected to sand milling and spraying, the particle size of the material did not meet the conditions defined in the present disclosure, and the specific surface area is small.

Table 1

|  | Particle size (μm) | | | | | |
|---|---|---|---|---|---|---|
|  | D50 | D0 | D10 | D90 | D99 | D100 |
| Example 1 | 6.97 | 0.52 | 1.93 | 15.2 | 24.8 | 35.2 |
| Example 2 | 7.25 | 0.68 | 2.13 | 17.9 | 26.5 | 38.2 |
| Example 3 | 6.21 | 0.43 | 1.78 | 14.1 | 22.6 | 32.9 |
| Example 4 | 6.55 | 0.47 | 1.85 | 14.6 | 23.4 | 33.6 |
| Example 5 | 8.38 | 0.75 | 2.25 | 19.5 | 27.8 | 39.7 |
| Example 6 | 7.39 | 0.72 | 2.18 | 18.6 | 27.3 | 38.9 |
| Example 7 | 5.25 | 0.36 | 1.67 | 13.2 | 21.0 | 31.5 |
| Comparative example 1 | 60.43 | 12.78 | 28.75 | 86.94 | 120.85 | 150.67 |
| Comparative example 2 | 75.90 | 22.35 | 45.12 | 100.29 | 168.92 | 197.83 |

Table 2

|  | Specific surface area ($m^2$/g) | pH |
|---|---|---|
| Example 1 | 50 | 6.78 |
| Example 2 | 41 | 6.54 |
| Example 3 | 55 | 6.65 |
| Example 4 | 52 | 6.71 |
| Example 5 | 37 | 6.75 |
| Example 6 | 39 | 6.63 |
| Example 7 | 57 | 6.85 |

(continued)

|  | Specific surface area (m²/g) | pH |
|---|---|---|
| Comparative example 1 | 0.78 | 6.75 |
| Comparative example 2 | 0.35 | 6.83 |

Experimental Example 2

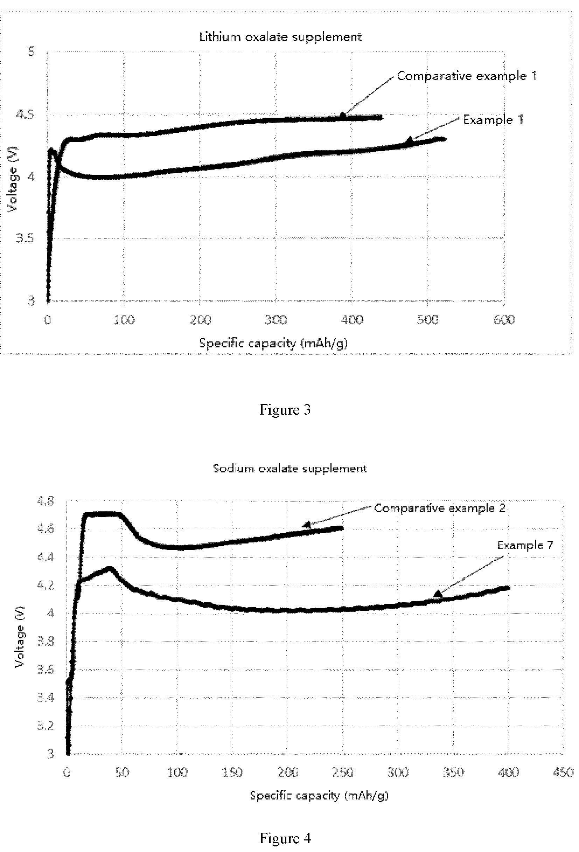

The additives obtained in Examples 1-6, Comparative Example 1 and Comparative Example 3 were respectively mixed with a conductive agent SP (carbon black) and a binder PVDF (polyvinylidene fluoride) in a mass ratio of 8:1:1 to form a positive electrode slurry, which was coated on an aluminum foil to form a positive electrode plate. Metal lithium was used as the negative electrode plate, Celgard 2400 microporous polypropylene membrane was used as the separator, and $LiPF_6$ (lithium hexafluorophosphate)/EC (ethylene carbonate)-DMC (dimethyl carbonate) was used as the electrolyte to assemble a lithium ion battery. The assembled lithium ion batteries were charged and tested respectively, and the charging test results were shown in Figure 3.

The additives obtained in Example 7 and Comparative Example 2 were respectively mixed with a conductive agent SP (carbon black) and a binder PVDF (polyvinylidene fluoride) in a mass ratio of 8:1:1 to form a positive electrode slurry, which was coated on an aluminum foil to form a positive electrode plate. Metallic sodium was used as the negative electrode plate, Celgard 2400 microporous polypropylene membrane was used as the separator, and $NaPF_6$ (sodium hexafluorophosphate)/EC (ethylene carbonate)-DMC (dimethyl carbonate) was used as the electrolyte to assemble a sodium ion battery. The assembled sodium ion batteries were charged and tested respectively, and the charging test results are shown in Figure 4.

The lithium oxalate prepared in Example 1 has a low decomposition voltage of 4.2V to 4.3V and a specific capacity of 520mAh/g. The lithium oxalate prepared in Comparative Example 1 has a high decomposition voltage of 4.3V to 4.5V and a specific capacity of 438mAh/g. The reason of Example 1 has excellent performance is that the material is nanometer-scale, and the lithium oxalate and the catalyst are fully and evenly mixed, which is beneficial for the catalyst to reduce the decomposition voltage of lithium oxalate and improve the capacity.

The sodium oxalate prepared in Example 7 has a low decomposition voltage of 4.2V to 4.3V and a specific capacity of 400mAh/g. The sodium oxalate prepared in Comparative Example 2 has a high decomposition voltage of 4.4V to 4.7V and a specific capacity of 249mAh/g. The reason of Example 2 has excellent performance is that the material is nanometer-scale, and the sodium oxalate and the catalyst are fully and evenly mixed, which is beneficial for the catalyst to reduce the decomposition voltage of the sodium oxalate and improve the capacity.

Industrial practicality

In summary, the present disclosure provides an additive for supplementing lithium or sodium and a preparation method and use thereof. The additive for supplementing lithium or sodium is low in decomposition voltage, high in specific capacity, small in particle size, and low in catalyst consumption. The preparation method is simple and easy to operate, and the obtained lithium or sodium supplement additive has excellent performance. The additives synthesized by the method are all nano-scale, and are mixed very evenly with the catalyst, with extremely low manufacturing cost, and are easy to mass-produce.

**Claims**

1. An additive for supplementing lithium or sodium, wherein the additive for supplementing lithium or sodium is mainly prepared from oxalic acid, a salt and a catalyst;

   wherein the salt comprises: a lithium salt or a sodium salt;
   the particle size distribution concentration ratio of the additive for supplementing lithium or sodium satisfies the following formula: $1 \leq (D90-D10)/D50 \leq 100$;
   the specific surface area of the additive for supplementing lithium or sodium is S, and S and D10, D50 and D90 of the additive for supplementing lithium or sodium satisfy the following formula: $1 \leq (S/((D90-D10)/D50) \leq 100$.

2. The additive for supplementing lithium or sodium according to claim 1, wherein D50 of oxalic acid, the salt, the catalyst and the additive for supplementing lithium or sodium are Da, Db, Dc and Dd respectively, and Da, Db, Dc and Dd satisfy the following formula: $0.5 \leq (Da+Db)/(Dc+Dd) \leq 200$.

3. The additive for supplementing lithium or sodium according to claim 1, wherein a mass ratio of oxalic acid, the salt and the catalyst is (170 to 180): (100 to 220): (5 to 30).

4. The additive for supplementing lithium or sodium according to claim 1, wherein the additive for supplementing lithium or sodium has a particle size of $0.01\mu m$ to $50\mu m$.

5. The additive for supplementing lithium or sodium according to claim 1, wherein the additive for supplementing lithium or sodium has a pH of less than 7.

6. The additive for supplementing lithium or sodium according to claim 1, wherein the additive for supplementing lithium or sodium has a bulk density of $0.5 \text{ g/cm}^3$ to $1.5 \text{ g/cm}^3$.

7. The additive for supplementing lithium or sodium according to claim 1, wherein the catalyst comprises at least one of: $LiCoO_2$, $LiNi_xCo_yMn_{1-x-y}O_2$, $LiFePO_4$, $LiMn_xFe_{1-x}O_4$, $LiMn_2O_4$, $LiNi_{0.5}Mn_{1.5}O_4$, lithium-rich manganese-based positive electrode, $NiO$, $MnO_2$, $Mn_3O_4$, $CoO$, $Co_3O_4$, $Fe_3O_4$, $MoO_3$, $WO_3$, $Nb_2O_5$, $Mo_2C$, $TaC$, $SiC$, $TiN$, $MoN$, $WN$, $TiB_2$, $WB$, Ketjen black, conductive carbon super-P, acetylene black, CNT, VGCF, polyaniline, polypyrrole, polythiophene and polypyridine; in the $LiNi_xCo_yMn_{1-x-y}O_2$, $x+y=1$, $0<x\leq1$, $0<y<1$; and in the $LiMn_xFe_{1-x}O_4$, $0\leq x\leq1$.

8. The additive for supplementing lithium or sodium according to claim 1, wherein the lithium salt comprises: $Li_2CO_3$ and/or $LiOH$.

9. The additive for supplementing lithium or sodium according to claim 1, wherein the sodium salt comprises at least one of $NaOH$, $Na_2CO_3$ or $NaHCO_3$.

10. A method for preparing the additive for supplementing lithium or sodium according to any one of claims 1 to 9, wherein the method comprises the following steps:
mixing a mixed solution containing oxalic acid, the salt and the catalyst and then sand milling and spray drying.

11. The method for preparing the additive for supplementing lithium or sodium according to claim 10, wherein the sand milling is carried out for a time period of 15 to 30 minutes.

12. The method for preparing the additive for supplementing lithium or sodium according to claim 10, wherein the sand milling has a material filling efficiency of 40% to 80%.

13. The method for preparing the additive for supplementing lithium or sodium according to claim 10, wherein the spray drying is carried out at a pressure of 0.2 to 1.0 MPa.

14. A positive electrode material, wherein the positive electrode material comprises the additive for supplementing lithium or sodium according to any one of claims 1 to 9.

15. A lithium ion or sodium ion battery, wherein the lithium ion or sodium ion battery comprises the positive electrode material according to claim 14.

Figure 1

Figure 2

Figure 3

Figure 4

## INTERNATIONAL SEARCH REPORT

| International application No. |
|---|
| **PCT/CN2024/089183** |

**A. CLASSIFICATION OF SUBJECT MATTER**

H01M 4/62(2006.01)i; H01M10/42(2006.01)i; H01M10/0525(2010.01)i; H01M10/054(2010.01)i

According to International Patent Classification (IPC) or to both national classification and IPC

**B. FIELDS SEARCHED**

Minimum documentation searched (classification system followed by classification symbols)

IPC: H01M4/-, H01M10/-

Documentation searched other than minimum documentation to the extent that such documents are included in the fields searched

Electronic data base consulted during the international search (name of data base and, where practicable, search terms used)

CNTXT, ENTXTC, CNKI, ISI_WEB OF SCIENCE: 补锂, 补钠, 锂补充, 钠补充, 补充锂, 补充钠, 草酸, 催化剂, 乙二酸, 粒度, 粒径, 比表面积, 砂磨, 研磨, 喷雾干燥, supplement, lithium, sodium, oxalic acid, catalyst, particle 2d size, diameter, specific 2d surface 2d area, sanding, spray, dry

**C. DOCUMENTS CONSIDERED TO BE RELEVANT**

| Category* | Citation of document, with indication, where appropriate, of the relevant passages | Relevant to claim No. |
|---|---|---|
| PX | CN 116454281 A (LIBAI NEW MATERIAL TECHNOLOGY (JIANGSU) CO., LTD.) 18 July 2023 (2023-07-18)<br>claims 1-10 | 1-15 |
| A | CN 114497553 A (HUIZHOU BYD BATTERY CO., LTD.) 13 May 2022 (2022-05-13)<br>description, embodiment 7 | 1-15 |
| A | CN 115395018 A (ZHONGCHUANG XINHANG TECHNOLOGY CO., LTD.) 25 November 2022 (2022-11-25)<br>entire description | 1-15 |
| A | CN 113394371 A (DONGGUAN TAFEL NEW ENERGY TECHNOLOGY CO., LTD. et al.) 14 September 2021 (2021-09-14)<br>entire description | 1-15 |
| A | CN 115954445 A (HUNAN TUOFENG NEW ENERGY CO., LTD.) 11 April 2023 (2023-04-11)<br>entire description | 1-15 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **10 July 2024** | **15 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/089183**

| C. | DOCUMENTS CONSIDERED TO BE RELEVANT | |
|---|---|---|
| Category* | Citation of document, with indication, where appropriate, of the relevant passages | Relevant to claim No. |
| A | US 2021126241 A1 (CONTEMPORARY AMPEREX TECHNOLOGY CO., LTD.) 29 April 2021 (2021-04-29)<br>entire description | 1-15 |

Form PCT/ISA/210 (second sheet) (July 2022)

## INTERNATIONAL SEARCH REPORT
### Information on patent family members

International application No.

**PCT/CN2024/089183**

| Patent document cited in search report | | | Publication date (day/month/year) | Patent family member(s) | | | Publication date (day/month/year) |
|---|---|---|---|---|---|---|---|
| CN | 116454281 | A | 18 July 2023 | None | | | |
| CN | 114497553 | A | 13 May 2022 | None | | | |
| CN | 115395018 | A | 25 November 2022 | None | | | |
| CN | 113394371 | A | 14 September 2021 | None | | | |
| CN | 115954445 | A | 11 April 2023 | None | | | |
| US | 2021126241 | A1 | 29 April 2021 | WO | 2020134777 | A1 | 02 July 2020 |
| | | | | EP | 3796432 | A1 | 24 March 2021 |
| | | | | EP | 3796432 | A4 | 13 October 2021 |
| | | | | EP | 3796432 | B1 | 02 November 2022 |
| | | | | ES | 2932152 | T3 | 13 January 2023 |
| | | | | PT | 3796432 | T | 29 November 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023104377951
- CN 114300680 A
- CN 114464909
- CN 110112475 A